# EUROPEAN PATENT SPECIFICATION

(11) **EP 0 915 120 B1**
(45) Date of publication and mention of the grant of the patent: **30.01.2002**
(21) Application number: 98308834.5
(22) Date of filing: 28.10.1998
(51) Int. Cl.: C08G 77/38, C08K 5/50, C08K 5/372, C08K 5/17, C08K 5/01, C08L 83/14

(54) **Method for termination of post cure in silicone elastomers**
Verfahren zur Beendigung der Nachhärtung in Silikonelastomeren
Méthode pour la terminaison de la post-réticulation dans des élastomères de silicone

(30) Priority: 05.11.1997 US 964546
(43) Date of publication of application: 12.05.1999
(73) Proprietor: DOW CORNING CORPORATION, Midland Michigan 48686-0994 (US)
(72) Inventor: Zhang, Shizhong, Midland, Michigan 48642 (US)
(74) Representative: Kyle, Diana

(56) References cited:
- US-A- 5 654 362

## Description

This invention is directed to methods for the quenching of post cure occurring in the thickening of silicone fluids and organic solvents with silicone elastomers.

In US-A 5,654,362, silicone elastomers are used in the thickening of silicone fluids and organic solvents. These elastomers are formed by a hydrosilylation reaction between a multifunctional ≡SiH siloxane and an alkene, preferably an α,ω-diene.

According to the above patent, one process that is used in making a silicone elastomer suitable in thickening a low viscosity silicone fluid or organic solvent is:
Step 1 - Gelation $\text{α,ω-diene + ≡Si-H Siloxane + Fluid + Catalyst → Elastomer}$
Step 2 - Shear & Swell $\text{Elastomer + More Fluid → Paste}$

When a crosslinked network is formed in such a reaction, we have determined that steric hindrance of the crosslinked structure prevents the reaction from reaching completion. This is due to the fact that a small amount of residual functionality will remain even after long reaction times and that unreacted functionalities will tend to meet each other when the elastomer is sheared and swollen. We have termed this phenomenon as "post cure". Because residual reactivity causes smooth, pasty products to further gel, the post cure phenomena should be eliminated in order to maintain a finer appearance and a more flowable rheology of the final paste product.

This is easily achieved, according to this invention, by quenching any residual ≡SiH functionality.

We have discovered that the post cure caused by residual crosslinking hydrosilylation reactions, which typically occur in the preparation of silicone elastomers, are conveniently terminated by introducing a ≡SiH quencher, such as a vinylsiloxane or vinylsilane, or (iii) an unsaturated organic compound which has a terminal alkyne group. In our method, and especially in the case of using a vinylsiloxane as the preferred quencher, the resulting product is not contaminated by any toxic ingredients. This is an important feature and advantage when such products are intended for use in the personal care or health care arenas.

Residual ≡SiH according to our invention can be quenched by molecules containing unsaturation such as alkene and alkyne. Because the internal crosslinking reaction is competing with our quenching reaction, the quenching reaction is usually performed faster than the crosslinking reaction. For example, using a large quantity of quencher is one way to increase the competition in favor of the quenching reaction, but this way may not be economical nor desirable for some applications.

Surprisingly, we have found that vinylsiloxanes and/or vinylsilanes can be used to completely terminate post cure problems. Vinylsiloxanes are preferred to react with ≡SiH over other alkenylsiloxanes. In our process of making a silicone paste, a vinylsiloxane is introduced at the shear and swell step. When this is done to our claimed invention, on-going reactions of residual functionalities are shifted to reactions between the incoming vinylsiloxane and the residual ≡SiH and the crosslinking reaction is terminated.

Preferably, a monovinylsiloxane is used because it is not capable of crosslinking. However, in the situation where moles of the quencher are in a large excess relative to ≡SiH, multivinylsiloxanes such as divinylsiloxane and trivinylsiloxane are also suitable for purposes of this invention.

Representative of some organosilicon monomers and polymers which are used as quenching agents according to our invention are silanes such as vinyl-t-butyldimethylsilane, vinyldiethylmethylsilane, vinylethyldimethylsilane, vinyltriethylsilane, vinyltrimethylsilane, divinyldimethylsilane and divinyltetramethyldisilane; and siloxanes such as vinylpentamethyldisiloxane, 1,3-divinyltetramethyldisiloxane, vinyltrisiloxane having the structure (CH₃)₃SiOSi(CH=CH₂)(CH₃)OSi(CH₃)₃, 1,5-divinylhexamethyltrisiloxane and divinylsiloxane oligomer having an average structure of (CH₂=CH)Me₂SiO(Me₂SiO)₈SiMe₂(CH=CH₂).

A schematic representation of a process according to this invention includes a vinylsiloxane which is depicted as competing with hexenyl groups as follows:

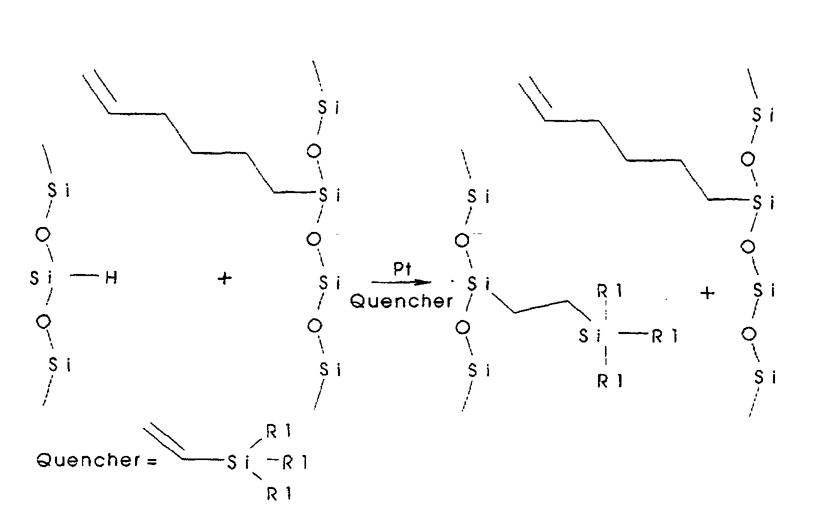

In another embodiment, post cure quenching is readily achieved using a post cure quenching agent other than a ligand or a silicon containing monomer or polymer. In this alternate embodiment, an unsaturated organic compound is used having a terminal alkyne group.

We have found that alkynes are more reactive than alkenes towards ≡Si-H in hydrosilylation reactions. Thus, when an alkyne is introduced at the shear and swell step, reactions of ≡Si-H to the alkenyl group will shift to an incoming alkyne. In this fashion, the crosslinking reaction will easily terminate.

Some examples of organic alkynes which are useful herein include acetylene, propyne, 1-butyne, 1-pentyne, 4,4-dimethyl-1-pentyne, 1-hexyne, 5-methyl-1-hexyne and 1-decyne.

It is also possible to use other unsaturated hydrocarbons such as alpha, omega-diynes of the formula CH≡C(CH₂)ₓC≡CH; or alpha, omega-ene-ynes of the formula CH₂=CH(CH₂)ₓC≡CH where x is 1-20. Some representative examples of alpha, omega-diynes are 1,3-butadiyne HC≡C-C≡CH and 1,5-hexadiyne (dipropargyl) HC≡C-CH₂CH₂-C≡CH. One example of a suitable alpha, omega-ene-yne is hexene-5-yne-1 CH₂=CHCH₂CH₂C≡CH.

Regardless of the type of post cure quenching agent selected for the purposes herein, an excess of post cure quenching agent is required. This not only increases the competition; but more importantly, it ensures consumption of all residual ≡Si-H groups. In particular, when a multifunctional vinyl siloxane or an alkyne is employed, the quantity of post cure quenching agent to be employed should be calculated on a molar basis rather than on an equivalent basis.

This invention is useful in any process involving silicone elastomers prepared by a crosslinking reaction between (A) ≡Si-H containing polysiloxanes and (B) an alkene, such as an alpha, omega-diene, in the presence of (C) a platinum group metal catalyst and (D) a solvent. Such elastomers are typically swollen with the molecular weight polysiloxanes under a shear force.

Usually, the ≡Si-H containing polysiloxane (A) is a polymer represented by the formula R₃SiO(R'₂SiO)ₐ(R"HSiO)_{b}SiR₃ designated herein as type A¹ and polymers of the formula HR₂SiO(R'₂SiO)_{c}SiR₂H or HR₂SiO(R'₂SiO)ₐ(R''HSiO)_{b}SiR₂H designated herein as type A². In these formulas, R, R' and R'', are alkyl groups with 1-6 carbon atoms; a is 0-250; b is 1-250 and c is 0-250. The molar ratio of compounds A²:A¹ is 0-20, preferably 0-5. In preferred embodiments, compounds of types A¹ and A² are used in the reaction; however, it is also possible to successfully conduct the reaction using only compounds of type A¹.

The term alkene (B) includes, and most preferably comprises, an alpha, omega-diene of the formula CH₂=CH(CH₂)ₓCH=CH₂ where x is 1-20. Representative alpha, omega-dienes are 1,4-pentadiene; 1,5-hexadiene; 1,6-heptadiene; 1,7-octadiene; 1,8-nonadiene; 1,9-decadiene; 1,11-dodecadiene; 1,13-tetradecadiene and 1,19-eicosadiene.

The term alkene is also intended to include siloxane monomers or polymers containing two or more terminal alkenyl groups; two or more pendant alkenyl groups; or two or more terminal and pendant groups. One suitable siloxane, for example, is tetramethyldivinylsiloxane.

These addition and crosslinking reactions require a catalyst (C) to effect reaction between the ≡SiH containing polysiloxane and the alpha, omega-diene. Suitable catalysts are the noble metals and, more particularly, the platinum group metals. Such metal catalysts are more completely described in US Patent 3,923,705. A preferred platinum catalyst is Karstedt's catalyst which is a platinum divinyl tetramethyl disiloxane complex, typically containing one weight percent of platinum, carried in a polydimethylsiloxane fluid or in an organic solvent such as toluene.

Another preferred platinum catalyst is a reaction product of chloroplatinic acid and an organosilicon compound containing terminal aliphatic unsaturation. It is further described in US Patent 3,419,593. These noble metal catalysts are normally used in amounts from 0.00001-0.5 part, per 100 weight parts of the ≡SiH containing polysiloxane. Preferably 0.00001-0.02 part, and most preferably 0.00001-0.002 part, are used in this invention.

The solvents (D) employed herein include organic compounds and silicone fluids of various types.

By "solvent", we mean (i) organic compounds, (ii) compounds containing a silicon atom, (iii) mixtures of organic compounds, (iv) mixtures of compounds containing a silicon atom, or (v) mixtures of organic compounds and compounds containing a silicon atom; used on an industrial scale to dissolve, suspend or change the physical properties of other materials.

The term silicone oil or fluid includes (i) low molecular weight linear and cyclic volatile methyl siloxanes, (ii) low molecular weight linear and cyclic volatile and non-volatile alkyl and aryl siloxanes, and (iii) low molecular weight linear and cyclic functional siloxanes. Most preferred, however, are low molecular weight linear and cyclic volatile methyl siloxanes (VMS).

VMS compounds correspond to the average unit formula (CH₃)ₐSiO_{(4-a)/2} in which a has an average value of two to three. These compounds contain siloxane units joined by ≡Si-O-Si≡ bonds. Representative units are monofunctional "M" units (CH₃)₃SiO_{1/2} and difunctional "D" units (CH₃)₂SiO_{2/2}.

The presence of trifunctional "T" units CH₃SiO_{3/2} results in the formation of branched linear or cyclic VMS. The presence of tetrafunctional "Q" units SiO_{4/2} results in the formation of branched linear or cyclic VMS.

Linear VMS have the formula (CH₃)₃SiO{(CH₃)₂SiO}_{y}Si(CH₃)₃. The value of y is 0-5. Cyclic VMS have the formula {(CH₃)₂SiO}_{z}. The value of z is 3-6. Preferably, these VMS have boiling points less than 250°C. and viscosities of 0.65-5.0 centistoke (mm²/s).

Representative linear VMS are hexamethyldisiloxane (MM) with a boiling point of 100°C., viscosity of 0.65 mm²/s and formula Me₃SiOSiMe₃; octamethyltrisiloxane (MDM) with a boiling point of 152°C., viscosity of 1.04 mm²/s and formula Me₃SiOMe₂SiOSiMe₃; decamethyltetrasiloxane (MD₂M) with a boiling point of 194°C., viscosity of 1.53 mm²/s and formula Me₃SiO (Me₂SiO)₂SiMe₃; dodecamethylpentasiloxane (MD₃M) with a boiling point of 229°C., viscosity of 2.06 mm²/s and formula Me₃SiO (Me₂SiO)₃SiMe₃; tetradecamethylhexasiloxane (MD₄M) with a boiling point of 245°C., viscosity of 2.63 mm²/s and formula Me₃SiO(Me₂SiO)₄SiMe₃; and hexadecamethylheptasiloxane (MD₅M) with a boiling point of 270°C., viscosity of 3.24 mm²/s and formula Me₃SiO(Me₂SiO)₅SiMe₃.

Representative cyclic VMS are hexamethylcyclotrisiloxane (D₃) a solid with a boiling point of 134°C. and formula {(Me₂)SiO}₃; octamethylcyclotetrasiloxane (D₄) with a boiling point of 176°C., viscosity of 2.3 mm²/s and formula {(Me₂)SiO}₄; decamethylcyclopentasiloxane (D₅) with a boiling point of 210°C., viscosity of 3.87 mm²/s and formula {(Me₂)SiO}₅; and dodecamethylcyclohexasiloxane (D₆) with a boiling point of 245°C., viscosity of 6.62 mm²/s and formula {(Me₂)SiO}₆.

Representative branched VMS are heptamethyl-3-{(trimethylsilyl)oxy}trisiloxane (M₃T) with a boiling point of 192°C., viscosity of 1.57 mm²/s and formula C₁₀H₃₀O₃Si₄; hexamethyl-3,3,bis {(trimethylsilyl)oxy} trisiloxane (M₄Q) with a boiling point of 222°C., viscosity of 2.86 mm²/s and formula C₁₂H₃₆O₄Si₅; and pentamethyl {(trimethylsilyl)oxy} cyclotrisiloxane (MD₃) with the formula C₈H₂₄O₄Si₄.

Our process also includes the use of low molecular weight linear and cyclic volatile and non-volatile alkyl and aryl siloxanes, represented respectively by the formulas R₃SiO(R₂SiO)_{y}SiR₃ and (R₂SiO)_{z}. R is an alkyl group of 1-6 carbon atoms or an aryl group such as phenyl. The value of y is 0-80, preferably 0-20. The value of z is 0-9, preferably 4-6. These polysiloxanes have viscosities generally in the range of 1-100 centistoke (mm²/s).

Other representative low molecular weight non-volatile polysiloxanes have the general structure: 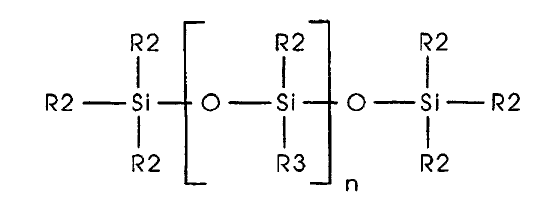 where n has a value to provide polymers with a viscosity in the range of 100-1,000 centistoke (mm²/sec).

R2 and R3 are alkyl radicals of 1-20 carbon atoms or an aryl group such as phenyl. Typically, the value of n is about 80-375. Illustrative polysiloxanes are polydimethylsiloxane, polydiethylsiloxane, polymethylethylsiloxane, polymethylphenylsiloxane and polydiphenylsiloxane.

Low molecular weight functional polysiloxanes useful herein are represented by acrylamide functional siloxane fluids, acrylate functional siloxane fluids, amide functional siloxane fluids, amino functional siloxane fluids, carbinol functional siloxane fluids, carboxy functional siloxane fluids, chloroalkyl functional siloxane fluids, epoxy functional siloxane fluids, glycol functional siloxane fluids, ketal functional siloxane fluids, mercapto functional siloxane fluids, methyl ester functional siloxane fluids, perfluoro functional siloxane fluids and silanol functional siloxanes.

Our invention is not limited to swelling silicone elastomers with only low molecular weight polysiloxanes. Other types of solvents, such as organic compounds, can swell the silicone elastomer. Thus, a single solvent or a mixture of solvents may be used.

In general, the organic compounds are aromatic hydrocarbons, aliphatic hydrocarbons, alcohols, aldehydes, ketones, amines, esters, ethers, glycols, glycol ethers, alkyl halides or aromatic halides. Representative of some common organic solvents are alcohols such as methanol, ethanol, 1-propanol, cyclohexanol, benzyl alcohol, 2-octanol, ethylene glycol, propylene glycol and glycerol; aliphatic hydrocarbons such as pentane, cyclohexane, heptane, Varnish Makers and Painters (VM&P) naphtha and mineral spirits; alkyl halides such as chloroform, carbon tetrachloride, perchloroethylene, ethyl chloride and chlorobenzene; amines such as isopropylamine, cyclohexylamine, ethanolamine and diethanolamine; aromatic hydrocarbons such as benzene, toluene, ethylbenzene and xylene; esters such as ethyl acetate, isopropyl acetate, ethyl acetoacetate, amyl acetate, isobutyl isobutyrate and benzyl acetate; ethers such as ethyl ether, n-butyl ether, tetrahydrofuran and 1,4-dioxane; glycol ethers such as ethylene glycol monomethyl ether, ethylene glycol monomethyl ether acetate, diethylene glycol monobutyl ether and propylene glycol monophenyl ether; ketones such as acetone, methyl ethyl ketone, cyclohexanone, diacetone alcohol, methyl amyl ketone and diisobutyl ketone; petroleum hydrocarbons such as mineral oil, gasoline, naphtha, kerosene, gas oil, heavy oil and crude oil; lubricating oils such as spindle oil and turbine oil; and fatty oils such as corn oil, soybean oil, olive oil, rape seed oil, cotton seed oil, sardine oil, herring oil and whale oil.

"Other" miscellaneous organic solvents can also be used, such as acetonitrile, nitromethane, dimethylformamide, propylene oxide, trioctyl phosphate, butyrolactone, furfural, pine oil, turpentine and m-creosol.

Further encompassed by the term solvent are volatile flavoring agents such as oil of wintergreen; peppermint oil; spearmint oil; menthol; vanilla; cinnamon oil; clove oil; bay oil; anise oil; eucalyptus oil; thyme oil; cedar leaf oil; oil of nutmeg; oil of sage; cassia oil; cocoa; licorice; high fructose corn syrup; citrus oils such as lemon, orange, lime and grapefruit; fruit essences such as apple, pear, peach, grape, strawberry, raspberry, cherry, plum, pineapple and apricot; and other useful flavoring agents including aldehydes and esters such as cinnamyl acetate, cinnamaldehyde, eugenyl formate, p-methylanisole, acetaldehyde, benzaldehyde, anisic aldehyde, citral, neral, decanal, vanillin, tolyl aldehyde, 2,6-dimethyloctanal and 2-ethyl butyraldehyde.

In addition, solvent also includes volatile fragrances such as natural products and perfume oils. Some representative natural products and perfume oils are ambergris, benzoin, civet, clove, leaf oil, jasmine, mate', mimosa, musk, myrrh, orris, sandalwood oil and vetivert oil; aroma chemicals such as amyl salicylate, amyl cinnamic aldehyde, benzyl acetate, citronellol, coumarin, geraniol, isobornyl acetate, ambrette and terpinyl acetate; and the various classic family perfume oils such as the floral bouquet family, the oriental family, the chypre family, the woody family, the citrus family, the canoe family, the leather family, the spice family and the herbal family.

Our basic process is a matter of combining the ≡SiH containing polysiloxane (A), the alkene (B), the solvent (D) and the catalyst (C); and then mixing these ingredients at room temperature until an elastomer is formed.

Additional amounts of silicone fluids or solvents are then added to the elastomer and the resulting mixture is subjected to shear force to form a paste. Any type of mixing and shearing equipment may be used to perform these steps such as a batch mixer, planetary mixer, single or multiple screw extruder, dynamic or static mixer, colloid mill, homogenizer, sonolator or any combination thereof.

Typically, our process uses approximately a 1:1 molar ratio of ≡Si-H containing polysiloxane and alkene, such as alpha, omega-diene. Useful products may also be prepared by conducting the above process with an excess of either the ≡Si-H containing polysiloxane or the alkene, but this is a less efficient use of these. The remainder of our claimed composition comprises silicone fluids or other solvents in amounts generally within the range of 65-98 percent by weight of the composition and preferably 80-98 percent by weight.

An essential feature of this invention is adding a post cure quenching agent (E), at or during the shear and swell step.

The silicone elastomer, silicone gel and silicone paste compositions of our invention have particular value in the personal care arena. Because of the unique volatility characteristics of the VMS component of these compositions, they can be used alone or blended with other cosmetic fluids, to form a variety of over-the-counter (OTC) personal care products.

Thus, they are useful as carriers in antiperspirants and deodorants, since they leave a dry feel and do not cool the skin upon evaporation. They are lubricious and will improve the properties of skin creams, skin care lotions, moisturizers, facial treatments such as acne or wrinkle removers, personal and facial cleansers, bath oils, perfumes, colognes, sachets, sunscreens, pre-shave and after-shave lotions, liquid soaps, shaving soaps and shaving lathers. They can be used in hair shampoos, hair conditioners, hair sprays, mousses, permanents, depilatories and cuticle coats, to enhance gloss and drying time and provide conditioning benefits.

In cosmetics, they will readily function as leveling and spreading agents for pigments in make-ups, color cosmetics, foundations, blushes, lipsticks, lip balms, eyeliners, mascaras, oil removers, color cosmetic removers and powders. They are useful as delivery systems for oil and water soluble substances such as vitamins. When incorporated into sticks, gels, lotions, aerosols and roll-ons, the compositions impart a dry, silky-smooth, payout.

In addition, the claimed compositions exhibit a . variety of advantageous and beneficial properties such as clarity, shelf stability and ease of preparation. Hence, they have wide application, but especially in antiperspirants, deodorants, in perfumes as a carrier and for conditioning hair.

Our silicone elastomers, gels and pastes have uses beyond the personal care arena, including their use as a filler or insulation material for electrical cable, a soil or water barrier for in-ground stabilization or as a replacement for epoxy materials used in coil-on-plug designs in the electronics industry.

They are also useful as carrier for crosslinked silicone rubber particles. In that application, (i) they allow ease of incorporation of the particles into such silicone or organic phases as sealants, paints, coatings, greases, adhesives, antifoams and potting compounds; and (ii) they provide for modifying rheological, physical or energy absorbing properties of such phases in either their neat or finished condition. Again, in such applications, a ligand may be a viable alternative as post cure quenching agent.

In addition, our silicone elastomers, gels and pastes are capable of functioning as carriers for pharmaceuticals, biocides, herbicides, pesticides and other biologically active substances; and can be used to incorporate water and water-soluble substances into hydrophobic systems. Examples of some water-soluble substances are salicylic acid, glycerol, enzymes and glycolic acid.

The following examples are set forth to further illustrate the invention in more detail.

### Example 1 - Quenching with Vinyltrisiloxane

A gel was made using the following ingredients:
(i) 51 g of an ≡SiH siloxane having an average structure represented by Me₃SiO(Me₂SiO)₉₃(MeHSiO)₆SiMe₃,
(ii) 264.2 g of decamethylcyclopentasiloxane (hereafter D5),
(iii) 1.82 g of 1,5-hexadiene, and
(iv) 0.058 g of Karstedt's catalyst with a Pt content of 0.51 %.

The above mixture was stirred in a capped container and heated at 55°C. until gelation. This gel was then heated in a 65-70°C. oven for three hours. The gel was next sheared and swollen with additional D5 to form a silicone paste containing 10.2 wt% of an elastomer. Vinyltrisiloxane (CH₃)₃SiOSi(CH=CH₂)(CH₃)OSi(CH₃)₃ in an amount of 200 mg was added (as the post curing quenching agent) and mixed with 91 g of the above silicone paste to yield a resulting product which remained pasty for an indefinite length of time.

Karstedt's catalyst is a preferred platinum catalyst herein and is more fully described in US Patents 3,715,334 and 3,814,730. Karstedt's catalyst is a platinum divinyl tetramethyl disiloxane complex, typically containing 0.5-1.0 weight percent of platinum, carried in a solvent such as toluene.

### Example 2 - Comparative Example

Another gel was prepared as in Example 1, sheared and swollen in D5 to form a silicone paste containing 10.2 wt% elastomer. No vinyltrisiloxane was added as a postcure quenching agent. The resulting paste was placed in a jar and gelled to a semisolid after 12 hours.

### Example 3 - Quenching with Vinylsiloxane Oligomer

A third gel was made according to Example 1, sheared and swollen in D5 to form a silicone paste containing 10.2 wt% elastomer. A vinylsiloxane oligomer with the average structure (CH₂=CH)Me₂SiO(Me₂SiO)₈SiMe₂(CH=CH₂), 260 mg, was mixed with 114 g of this paste. No further gelation was observed and the product remained pasty.

### Example 4 - Quenching with Terminal Alkyne

A fourth gel was prepared according to Example 1, sheared and swollen in D5 to form a silicone paste containing 10.2 wt% elastomer. 1-Decyne CH₃(CH₂)₇C≡CH, 120 mg, was mixed with 100 g of this paste. No further gelation was observed and the product remained pasty.

## Claims

1. A method of thickening organic solvents comprising combining and reacting:
(A) a ≡Si-H containing polysiloxane of formula R₃SiO(R'₂SiO)ₐ(R''HSiO)_{b}SiR₃ and optionally a ≡Si-H containing polysiloxane of formula HR₂SiO(R'₂SiO)_{c}SiR₂H or a ≡Si-H containing polysiloxane of formula HR₂SiO(R'₂SiO)ₐ(R"HSiO)bSiR₂H where R, R' and R'' are alkyl groups of 1-6 carbon atoms; a, is 0-250; b is 1-250; and c is 0-250;
(B) an alkene;
(C) a platinum group metal catalyst; and
(D) an organic solvent until said elastomer is formed by crosslinking and addition of ≡Si-H across double bonds in said alkene; adding additional amounts of solvent to the silicone elastomer and adding an effective amount of a post cure quenching agent (E) selected from vinylsiloxanes, vinylsilanes, and unsaturated organic compounds having a terminal alkyne group; and subjecting the solvent, the post cure quenching agent and the elastomer to shear force until a paste is formed.

2. The method of claim 1 wherein said alkene is an alpha, omega-diene of formula CH₂=CH(CH₂)ₓCH=CH₂ where x is 1-20.

3. The method of claim 1 or 2, wherein said solvent is selected from the group consisting of (i) organic compounds, (ii) compounds containing a silicon atom, (iii) mixtures of organic compounds, (iv) mixtures of compounds containing a silicon atom, and (v) mixtures of organic compounds and compounds containing a silicon atom.

4. The method according to any of claims 1 to 3, in which the post cure quenching agent (E) is a ≡SiH quencher selected from vinyl-t-butyldimethylsilane, vinyldiethylmethylsilane, vinylethyldimethylsilane, vinyltriethylsilane, vinyltrimethylsilane, divinyldimethylsilane, divinyltetramethyldisilane, vinylpentamethyldisiloxane, 1,3-divinyltetramethyldi-siloxane, vinyltrisiloxane (CH₃)₃SiOSi(CH=CH₂)(CH₃)OSi(CH₃)₃, 1,5-divinylhexamethyltrisiloxane and divinylsiloxane oligomer (CH₂=CH)Me₂SiO(Me₂SiO)₈SiMe₂(CH=CH₂).

5. A method according any of claims 1 to 3, in which the post cure quenching agent is an unsaturated organic compound selected from acetylene, propyne, 1-butyne, 1-pentyne, 4,4-dimethyl-1-pentyne, 1-hexyne, 5-methyl-1-hexyne, 1-decyne, 1,3-butadiyne, 1,5-hexadiyne and hexene-5-yne-1.

6. A personal care product containing the paste obtained by the method of any of claims 1 to 5 selected from antiperspirants, deodorants, skin creams, skin care lotions, moisturizers, acne removers, wrinkle removers, facial cleansers, bath oils, perfumes, colognes, sachets, sunscreens, pre-shave lotions, after-shave lotions, liquid soaps, shaving soaps, shaving lathers, hair shampoos, hair conditioners, hair sprays, mousses, permanents, depilatories, cuticle coats, make-ups, color cosmetics, foundations, blushes, lipsticks, lip balms, eyeliners, mascaras, oil removers and cosmetic removers.

7. A product containing the paste obtained by the method of any of claims 1 to 5 and a material selected from crosslinked silicone rubber particles, pharmaceuticals, biocides, herbicides, pesticides, water and water-soluble substances.

8. Use of the personal care product of claim 6 by applying to the hair, skin or underarm.

9. A method of modifying rheological, physical or energy absorbing properties, of silicone or organic phases selected from sealants, paints, coatings, greases, adhesives, antifoams and potting compounds, comprising incorporating therein the paste obtained by the method of any of claims 1 to 5 containing crosslinked silicone rubber particles.

10. A method of filling or insulating an electrical cable comprising incorporating therein the paste obtained by the method of any of claims 1 to 5.

11. A method of stabilizing in-ground soil or water barriers comprising incorporating into soil the paste obtained by the method of any of claims 1 to 5.

## Patentansprüche

1. Verfahren zur Verdickung von organischen Lösungsmitteln, umfassend Vereinigen und Umsetzen von:
(A) einem ≡Si-H-haltigen Polysiloxan der Formel R₃SiO(R'₂SiO)ₐ(R"HSiO)_{b}SiR₃ und wahlweise einem ≡Si-H-haltigen Polysiloxan der Formel HR₂SiO(R'₂SiO)_{c}SiR₂H oder einem ≡Si-H-haltigen Polysiloxan der Formel HR₂SiO(R'₂SiO)ₐ(R"HSiO)_{b}SiR₂H, worin R, R' und R" Alkylgruppen mit 1 bis 6 Kohlenstoffatomen sind; a gleich 0 bis 250 ist; b gleich 1 bis 250 ist und c gleich 0 bis 250 ist;
(B) einem Alken;
(C) einem Platingruppenmetallkatalysator und
(D) einem organischen Lösungsmittel, bis ein Elastomer durch Vernetzung und Addition der ≡Si-H an Doppelbindungen in diesem Alken gebildet wird; Hinzufügen von weiteren Mengen von Lösungsmittel zu dem Siliconelastomer und Hinzufügen einer wirksamen Menge eines Abschreckungsmittels (E) zur Beendigung der Nachhärtung, ausgewählt aus Vinylsiloxanen, Vinylsilanen und ungesättigten organischen Verbindungen mit endständigen Alkingruppen: und Aussetzen des Lösungsmittels, des Abschreckungsmittels zur Beendigung der Nachhärtung und des Elastomers an Scherkräfte, bis eine Paste gebildet wird.

2. Verfahren nach Anspruch 1, worin dieses Alken ein alpha,omega-Dien der Formel CH₂=CH(CH₂)ₓCH=CH₂ ist, worin x gleich 1 bis 20 ist.

3. Verfahren nach Anspruch 1 oder 2, worin dieses Lösungsmittel ausgewählt ist aus der Gruppe bestehend aus (i) organischen Verbindungen, (ii) Verbindungen, die ein Siliciumatom enthalten, (iii) Mischungen von organischen Verbindungen, (iv) Mischungen von Verbindungen, die ein Siliciumatom enthalten, und (v) Mischungen von organischen Verbindungen und Verbindungen, die ein Siliciumatom enthalten.

4. Verfahren nach einem der Ansprüche 1 bis 3, in dem das Abschreckmittel (E) zur Beendigung der Nachhärtung ein ≡Si-H-Abschreckmittel ist, ausgewählt aus Vinyl-t-butyldimethylsilan, Vinyldiethylmethylsilan, Vinylethyldimethylsilan, Vinyltriethylsilan, Vinyltrimethylsilan, Divinyldimethylsilan, Divinyltetramethyldisilan, Vinylpentamethyldisiloxan, 1,3-Divinyltetramethyldisiloxan, Vinyltrisiloxan (CH₃)₃SiOSi(CH=CH₂)(CH₃)OSi(CH₃)₃, 1,5-Divinylhexamethyltrisiloxan und Divinylsiloxanoligomer (CH₂=CH)Me₂SiO(Me₂SiO)₈SiMe₂(CH=CH₂).

5. Verfahren nach einem der Ansprüche 1 bis 3, in dem das Abschreckmittel zur Beendigung der Nachhärtung eine ungesättigte organische Verbindung, ausgewählt aus Acetylen, Propin, 1-Butin, 1-Pentin, 4,4-Dimethyl-1-pentin, 1-Hexin, 5-Methyl-1-hexin, 1-Decin, 1,3-Butadiin, 1,5-Hexadiin und Hexen-5-in-1, ist.

6. Körperpflegeprodukt, das die Paste enthält, die nach dem Verfahren aus einem der Ansprüche 1 bis 5 erhalten wurde, ausgewählt aus Antiperspirationsmitteln, Deodorantien, Hautcremes, Hautpflegelotionen, feuchtigkeitsspendenden Mitteln, Akneentfernungsmitteln, Faltenentfernungsmitteln, Gesichtsreinigern, Badeölen, Parfums, Eau de Colognes, Sachets, Sonnenschutzmitteln, Pre-Shave-Lotionen, After-Shave-Lotionen, Flüssigseifen, Rasierseifen, Rasierschäumen, Haarshampoos, Haarkonditionierungsmitteln, Haarsprays, Schäumen, Dauerwellpräparaten, Enthaarungsmitteln, Nagelhautabdeckern, Make-Ups, dekorativen Kosmetika, Grundierungen, Rouges, Lippenstiften, Lippenpflegestiften, Lidstiften, Wimperntuschen, Ölentfernern und Kosmetikentfernungspräparaten.

7. Produkt, das die Paste, die nach dem Verfahren aus einem der Ansprüche 1 bis 5 erhalten wurde, und einen Stoff enthält, ausgewählt aus vernetzten Siliconkautschukteilchen, Arzneimitteln, Bioziden, Herbiziden, Pestiziden, Wasser und wasserlöslichen Substanzen.

8. Verwendung des Körperpflegeprodukts aus Anspruch 6 durch Auftragen auf das Haar, die Haut oder die Achselhöhle.

9. Verfahren zur Modifizierung der rheologischen, physikalischen oder energieabsorbierenden Eigenschaften von Silicon oder organischen Phasen, ausgewählt aus Dichtmitteln, Farben, Beschichtungsmitteln, Fetten, Klebstoffen, Anti-Schaummitteln und Einbettmasssen, umfassend das darin Einfügen der Paste, die nach dem Verfahren aus einem der Ansprüche 1 bis 5 erhalten wird und vernetzte Siliconkautschukteilchen enthält.

10. Verfahren zur Füllung oder Isolierung eines elektrischen Kabels, umfassend Einbringen darin der Paste, die nach dem Verfahren nach einem der Ansprüche 1 bis 5 erhalten wird.

11. Verfahren zur Stabilisierung von Erdreich- oder Wassersperren im Boden, umfassend Einarbeitung der Paste, die nach dem Verfahren aus einem der Ansprüche 1 bis 5 erhalten wird, in das Erdreich.

## Revendications

1. Méthode d'épaississement de solvants organiques, comprenant la combinaison et la mise en réaction:
(A) d'un polysiloxane contenant ≡Si-H, de formule R₃SiO(R'₂SiO)ₐ(R''HSiO)_{b}SiR₃ et, éventuellement, un polysiloxane contenant ≡Si-H, de formule HR₂SiO(R'₂SiO)_{c} SiR₂H ou un polysiloxane contenant ≡Si-H de formule HR₂SiO(R'₂SiO)ₐ(R''HSiO)_{b}SiR₂H, formules dans lesquelles R, R' et R' ' sont des groupes alkyles de 1 à 6 atomes de carbone ; a est égal à 0-250 ; b est égal à 1-250 et c est égal à 0-250 ;
(B) un alcène ;
(C) un catalyseur d'un métal du groupe du platine ; et
(D) un solvant organique, jusqu'à ce que ledit élastomère soit formé par réticulation et addition de ≡Si-H à travers des doubles liaisons dans ledit alcène ; ajout de quantités supplémentaires de solvant à l'élastomère de silicone et ajout d'une quantité efficace d'un agent d'extinction de post-réticulation (E) choisi parmi les vinylsiloxanes, les vinylsilanes et les composés organiques insaturés possédant un groupe alcyne terminal ; et exposition du solvant, de l'agent d'extinction de post-réticulation et de l'élastomère, à une force de cisaillement jusqu'à formation d'une pâte.

2. Méthode selon la revendication 1, dans laquelle ledit alcène est un alpha, oméga-diène de formule CH₂=CH(CH₂)ₓCH=CH₂ dans laquelle x est 1 - 20.

3. Méthode selon la revendication 1 ou 2, dans laquelle ledit solvant est choisi dans le groupe constitué de (i) composés organiques , (ii) composés contenant un atome de silicium, (iii) mélanges de composés organiques, (iv) mélanges de composés contenant un atome de silicium et (v) mélanges de composés organiques et de composés contenant un atome de silicium.

4. Méthode selon l'une quelconque des revendications 1 à 3, dans laquelle l'agent d'extinction de post-réticulation (E) est un agent d'extinction ≡SiH choisi parmi le vinyl-t-butyldiméthylsilane, le vinyldiéthylméthylsilane, le vinyléthyldiméthylsilane, le vinyltriéthylsilane, le vinyltriméthylsilane, le divinyldiméthylsilane, le divinyltétraméthyldisilane, le vinylpentaméthyldisiloxane, le 1,3-divinyltétraméthyldisiloxane, le vinyltrisiloxane (CH₃)₃SiOSi(CH=CH₂) (CH₃)OSi(CH₃)₃, le 1,5-divinylhexaméthyltrisiloxane et l'oligomère de divinylsiloxane (CH₂=CH) Me₂SiO (Me₂SiO)₈SiMe₂(CH=CH₂).

5. Méthode selon l'une quelconque des revendications 1 à 3, dans laquelle l'agent d'extinction de post-réticulation est un composé organique insaturé choisi parmi l'acétylène, le propyne, le 1-butyne, le 1-pentyne, le 4,4-diméthyl-1-pentyne, le 1-hexyne, le 5-méthyl-1-hexyne, le 1-décyne, le 1,3-butadiyne, le 1,5-hexadiyne et l'hexène-5-yne-1.

6. Produit de soins pour le corps contenant la pâte obtenue par la méthode de l'une quelconque des revendications 1 à 5, choisi parmi les anti-transpirants, les déodorants, les crèmes pour la peau, les lotions de soins pour la peau, les hydratants, les produits anti-acné, les anti-rides, les nettoyants pour le corps et le visage, les huiles pour le bain, les parfums, les eaux de Cologne, les sachets, les crèmes solaires, les lotions de pré-rasage, les lotions d'après rasage, les savons liquides, les savons à barbe, les mousses à raser, les shampooings, les après-shampooings, les laques, les mousses, les produits pour permanentes, les crèmes dépilatoires, les pâtes anti-cuticules, le maquillage, les cosmétiques colorants, les fonds de teint, les fards, les rouges à lèvres, les pommades pour les lèvres, les eye-liners, les mascaras, les dégraissants et les produits démaquillants.

7. Produit contenant la pâte obtenue par la méthode de l'une quelconque des revendications 1 à 5 et matière choisie parmi des particules de caoutchouc de silicone réticulé, produits pharmaceutiques, biocides, herbicides, pesticides, eau et substances hydrosolubles.

8. Utilisation du produit de soins pour le corps selon la revendication 6, par application sur les cheveux, la peau ou l'aisselle.

9. Méthode de modification des propriétés rhéologiques, physiques ou d'absorption d'énergie, de phases silicones ou organiques choisies parmi les matériaux d'étanchéité, peintures, revêtements, graisses, adhésifs, anti-mousses, et composés d'empotage comprenant, incorporée dans ces derniers, la pâte obtenue par la méthode de l'une quelconque des revendications 1 à 5, contenant des particules de caoutchouc de silicone réticulé.

10. Méthode de remplissage ou d'isolation d'un câble électrique comprenant l'incorporation dans celui-ci de la pâte obtenue par la méthode de l'une quelconque des revendications 1 à 5.

11. Méthode de stabilisation de barrières de sol ou d'eau en sous-sol comprenant l'incorporation dans le sol de la pâte obtenue par la méthode de l'une quelconque des revendications 1 à 5.
